# EUROPÄISCHE PATENTSCHRIFT

(11) **EP 2 981 179 B1**
(45) Veröffentlichungstag und Bekanntmachung des Hinweises auf die Patenterteilung: **04.03.2020**
(21) Anmeldenummer: 14701924.4
(22) Anmeldetag: 08.01.2014
(51) Int. Cl.: A23L 27/10, C07C 47/58, C12P 7/24

(54) **STOFFGEMISCHE ENTHALTEND VANILLIN UND VANILLYLVANILLAT**
COMPOSITIONS COMPRISING VANILLIN AND VANILLYL VANILLATE
COMPOSITIONS COMPRENANT VANILLINE ET VANILLYL VANILLATE

(30) Priorität: 27.02.2013 EP 13157041
(43) Veröffentlichungstag der Anmeldung: 10.02.2016
(73) Patentinhaber: Symrise AG, 37603 Holzminden (DE)
(72) Erfinder: KINDEL, Günter, 37671 Höxter (DE); GROSS, Egon-Eduard, 37603 Holzminden (DE); HILMER, Jens-Michael, 37603 Holzminden (DE)
(74) Vertreter: Fabry, Bernd
(86) Internationale Anmeldenummer: PCT/EP2014/050248
(87) Internationale Veröffentlichungsnummer: WO 2014/131532

(56) Entgegenhaltungen:
- EP-A1- 2 353 403
- WO-A1-2006/079089

## Beschreibung

### GEBIET DER ERFINDUNG

Die Erfindung befindet sich auf dem Gebiet der Aromastoffe und betrifft Stoffgemische, die in ihrer geschmacklichen Beurteilung mit natürlicher Vanille weitgehend identisch sind.

### STAND DER TECHNIK

Vanillin ist ein wichtiger, in der Nahrungs- und Genussmittel-Industrie viel verwendeter Aromastoff. Bislang wird es hauptsächlich aus dem Lignin der Sulfitlaugen, gelegentlich auch durch Oxidation von Eugenol oder Isoeugenol auf chemischem Wege hergestellt. Das auf diese Weise erhaltene Vanillin hat jedoch den Nachteil, dass es im lebensmittelrechtlichen Sinne kein Naturstoff ist und deshalb auch nicht als natürlicher Aromastoff bezeichnet werden darf.

Der natürliche Aromastoff Vanillin ist bisher nur durch Extraktion von Vanilleschoten erhältlich; das auf diese Weise erhaltene natürliche Vanillin ist jedoch sehr teuer. Natürliche Aromastoffe sind chemisch definierte Stoffe mit Aromaeigenschaften, gewonnen durch geeignete physikalische Verfahren (einschließlich Destillation und Extraktion mit Lösungsmitteln), durch enzymatische oder mikrobiologische Verfahren aus Ausgangsstoffen pflanzlicher oder tierischer Herkunft, die als solche verwendet oder mittels herkömmlicher Lebensmittelzubereitungsverfahren (einschließlich Trocknen, Rösten, Fermentieren) für den menschlichen Verzehr aufbereitet werden.

Verschiedene andere Verfahren zur Herstellung von natürlichem Vanillin mit unterschiedlichen Mikroorganismen und Enzymen sind in der Zwischenzeit veröffentlicht worden, liefern aber bislang aufgrund der nur recht geringen Ausbeuten bzw. Konzentrationen keine Produkte auf dem Markt. Beispiele für einschlägige Verfahrens des Stands der Technik finden sich etwa in den Druckschriften EP 0405197 A1 (HAARMANN & REIMER), EP 0453368 A1 (PERNOD RICARD), EP 0542348 A1 (QUEST), EP 0885968 A1 (GIVAUDAN) oder US 5,128,253 (GEN FOODS).

Aus der EP 0761817 B1 (HAARMANN & REIMER) ist ebenfalls ein Verfahren zur Herstellung von Vanillin bekannt, bei dem man Ferulasäure aus Eugenol in Gegenwart von bestimmten Bakterien zu Vanillin fermentieren kann. Die Ausbeuten sind dabei deutlich höher als aus anderen Verfahren bekannt, jedoch ist die sensorische Beurteilung der Fermentationsprodukte unbefriedigend.

Das Dokument WO 2006/079089 A1 betrifft ein Duftstoffabgabesystem, das folgende Komponenten umfasst: 1) Wasser unlösliche Trägerpartikel, die auf ihrer Oberfläche Silanolgruppen tragen, 2) mindestens ein Polymer, das positiv geladene funktionelle Gruppen trägt, und 3) als dritte Komponente ein Duftstoff, der auf oder in den Trägerpartikeln absorbiert wird. Unter den vielen genannten Duftstoffen, die in Frage kommen können, werden Vanillin und Vanillylvanillat genannt.

Das Dokument EP 2 353 403 A1 betrifft die Verwendung von Deoxyhesperetindihydrochalkon oder eines Salzes des Deoxyhesperetindihydrochalkons oder einer Mischung umfassend oder bestehend aus mehreren Salzen des Deoxyhesperetindihydrochalkons oder einer Mischung umfassend oder bestehend aus Deoxyhesperetindihydrochalkon und einem Salz des Deoxyhesperetindihydrochalkons oder mehrere Salze des Deoxyhesperetindihydrochalkons, zum Beeinflussen der Stärke von Geschmackseindrücken süß schmeckender, unangenehm, insbesondere bitter, schmeckender oder sowohl süß als auch unangenehm, insbesondere bitter, schmeckender Stoffe oder Stoffgemische. Konkret beansprucht werden auch Zusammensetzungen, die neben Deoxyhesperetindihydrochalkon auch einen oder mehrere süß schmeckende Stoffe, unter anderem Vanillin, beinhalten.

Nachdem es bislang nicht in zufriedenstellendem Umfang möglich ist, Vanillin auf synthetischem, mikrobiellem Wege in einer geschmacklichen Qualität herzustellen, die dem von natürlichem Vanillin zumindest weitgehend entspricht, hat die Aufgabe der vorliegenden Erfindung hat somit darin bestanden, eine alternative Lösung anzubieten, eine synthetisches Aroma mit hoher Übereinstimmung mit dem Geschmacksprofil von natürlicher Vanille zur Verfügung zu stellen.

### BESCHREIBUNG DER ERFINDUNG

Gegenstand der Erfindung sind Stoffgemische, enthaltend
(a) Vanillin und
(b) Vanillylvanillat
wobei die Stoffgemische die Komponenten (a) und (b) im Gewichtsverhältnis von etwa 25:75 bis etwa 75:25 enthalten.

Überraschenderweise wurde gefunden, dass synthetisches Vanillin und synthetisches Vanillylvanillat im Gegensatz zu ihrem Geschmacksprofil als Einzelsubstanzen ein synergistisches Verhalten zeigen und innerhalb breiter Grenzen der natürlichen Vanille sehr nahe kommen.

### Vanillin

Vanillin kann grundsätzlich in an sich bekannter Weise durch Fermentation unterschiedlicher Einsatzstoffe, wie beispielsweise Reis, Mais oder Getreide über die Zwischenstufe der Ferulasäure gewonnen werden. Vorzugsweise gelangt aber eine Vanillinqualität zum Einsatz, die auf Basis von Maiskleie gewonnen wird und dies insbesondere gemäß nachfolgender Beschreibung.

Das bevorzugte Verfahren zur Herstellung von Vanillin umfasst dabei die folgenden Schritte, bei dem man
(a) zunächst einen Rückstand aus der Maisverarbeitung mit Alkalien oder esterspaltenden Enzymen behandelt und dabei das darin enthaltende γ-Oryzanol zu Ferulasäure und Sterolen hydrolysiert,
(b) die Ferulasäure aus der Mischung isoliert,
(c) die isolierte Ferulasäure zusammen mit Mikroorganismen vom Typ *Amycolatopsis sp.* DSM 9991 und/oder sp. 9992 in einem Kulturmedium fermentiert, und
(d) nach Beendigung der Reaktion das gebildete Vanillin isoliert.

Obschon grundsätzlich bekannt ist, dass synthetisch gewonnenes Vanillin je nach Ausgangsstoff und Verfahren infolge der Nebenbestandteile eine etwas andere Geschmacksnote besitzt, wurde überraschend gefunden, dass Ferulasäure, die aus Mais gewonnen wird in Kombination mit einem Fermentationsverfahren, das mit speziellen Bakterien vom Typ der Actinomyceten im Ergebnis zu einer Vanillinqualität führt, die zusammen mit Vanillylvanillat besonders gut harmoniert.

Die Gewinnung von Ferulasäure aus Rückständen der Reis- oder auch Maisproduktion grundsätzlich bekannt. Beispielsweise wird in der Monographie von Berger "Flavours & Fragrances" im Kapitel 23.4 "Microbial Flavour Production" im Schaubild auf Seite 532 erläutert, dass durch Behandlung dieser Ausgangsstoffe mit geeigneten Esterasen Ferulasäure gewonnen werden kann, die dann mit nicht näher definierten Amycolatopsis-Stämmen in Vanillin umgewandelt werden kann.

Stärkehaltige Pflanzen, wie Kartoffeln, Mais und vor allem Reis, enthalten in unterschiedlichen Mengen γ-Oryzanol, 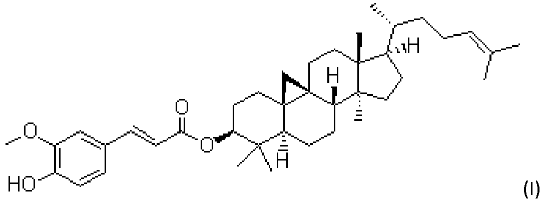 einen Ester der Ferulasäure mit unterschiedlichen Phytosterolen, wie er in der Strukturformel (I) oben exemplarisch dargestellt ist. Durch Behandlung mit Alkalien oder esterspaltenden Enzymen wird der Ester hydrolysiert bzw. verseift und die Ferulasäure freigesetzt. Vorzugsweise erfolgt dieser Schritt als enzymatische Hydrolyse. Als Enzyme kommen für diesen Zweck bevorzugt Ferulasäureesterasen in Frage.

Die Hydrolyse bzw. Verseifung kann dabei in an sich bekannter Weise erfolgen, wie dies beispielsweise für die Herstellung von Ferulasäure aus Reiskleie aus der EP 0503650 B1 (Wakayama) bekannt ist. Dabei kommen etwa Natrium- oder Kaliumhydroxid oder auch entsprechende andere basische Alkalisalze zum Einsatz. Da die Rückstände im Wesentlichen wasserunlöslich sind empfiehlt es sich, ein geeignetes Lösungsmittel, beispielsweise Ethanol mitzuverwenden. Die alkalische Hydrolyse wird in der Regel drucklos bei Temperaturen im Bereich von 90 bis 100 °C und pH-Werten von wenigstens 10 durchgeführt, während die enzymatische Variante deutlich niedrigere Temperaturen und ein eher neutrales Milieu erlaubt, die dem Aktivitätsoptimum der Esterasen entsprechen und typisch bei 28 bis 35 °C und pH 7 bis 8 liegen. Die Reaktionszeiten liegen in der Regel bei etwa 5 bis 24, vorzugsweise etwa 8 bis 20 und insbesondere etwa 10 bis 12 h.

Nach der Hydrolyse liegt die Ferulasäure als Salz vor. Durch Zugabe beispielsweise von verdünnter Schwefelsäure und Einstellen eines pH-Wertes von etwa 2 wird die Säure freigesetzt und fällt bei Temperaturen unterhalb von 30 °C aus. Das Präzipitat kann abfiltriert, gewaschen und gegebenenfalls aus heißem Wasser umkristallisiert werden, wobei die Säure in praktisch reinem Zustand erhalten wird und ohne weitere Aufreinigung in die Fermentation eingesetzt werden kann.

Bei der anschließenden Fermentation kommt es zur Umwandlung von Ferulasäure (II) zu Vanillin (III):

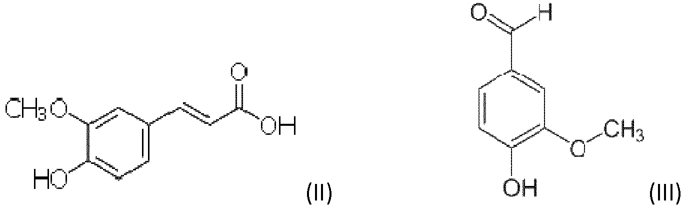

Die Fermentation wird in Gegenwart von Mikroorganismen des Typs *Amycolatopsis species* aus der Gattung Pseudonocardia durchgeführt; entsprechende Stämme sind bei der Deutschen Sammlung für Mikroorganismen und Zellkulturen GmbH in Braunschweig unter den Nummern DSM 9991 und DSM 9992 hinterlegt. Aufgrund der chemotaxonomischen Untersuchungsergebnisse wurden beide Isolate der Gattung Amycolatopsis (Familie Pseudonocardiaceä) zugeordnet. Die Identifizierung basierte auf folgenden fünf Merkmalen: 1. Diagn. Aminosäure des Peptidoglycans: meso-Diaminopimelinsäure; 2. Diagn. Zucker aus Ganzzellhydrolysaten: Arabinose und Galaktose; 3. Mycolsäuren: fehlen; 4. Menachinone: MK - 9 (H4); 5. Fettsäuremuster: Iso/Anteiso- + 10methyl-verzweigte gesättigte und ungesättigte Fettsäuren plus 2-Hydroxy-Fettsäuren. Dieses Fettsäuremuster ist diagnostisch für Vertreter der Gattung Amycolatopsis. Da DSM 9991 und DSM 9992 auch das typische Erscheinungsbild der Vertreter der Gattung Amycolatopsis zeigen - beige bis gelbes Substratmycel und auf einigen Medien auch ein feines weißes Luftmycel - wird die Zuordnung von DSM 9991 und DSM 9992 zur Gattung Amycolatopsis zusätzlich durch die Morphologie unterstützt.

Die Mikroorganismen können in einem üblichen Kulturmedium in für die Kultivierung von Mikroorganismen üblicher Weise kultiviert werden. Das Substrat kann zu Beginn der Inkubation, während oder nach Abschluss des Wachstums auf einmal oder über einen längeren Zeitraum verteilt zugegeben werden. Die Menge an Ferulasäure wird dabei vorteilhaft so bemessen, dass die Konzentration der Verbindung in der Kulturbrühe 80 g/l, vorzugsweise 15 g/l nicht überschreitet. Der Verlauf der Umsetzung kann durch Bestimmung des Ausgangsmaterials und des Produkts in der Kulturbrühe mittels Hochdruckflüssigkeitschromatographie verfolgt werden. Nachdem die optimale Menge an Vanillin entstanden ist, wird dieses durch bekannte physikalische Verfahren wie Extraktion, Destillation oder Chromatographie aus der Kulturbrühe isoliert. Das so erhaltene Rohprodukt kann durch weitere Schritte gereinigt werden.

Die Kultivierung der Mikroorganismen kann in synthetischen, halbsynthetischen oder komplexen Kulturmedien erfolgen. Diese Kulturmedien enthalten vorzugweise Kohlenstoffquellen, Stickstoffquellen, anorganische Salze und gegebenenfalls Spurenelemente und Vitamine. Als Kohlenstoffquellen können z.B. Zucker wie Glucose, Zuckeralkohole wie Glycerin oder Mannit, organische Säuren wie Zitronensäure oder komplexe Gemische wie Malzextrakt, Hefeextrakt, Casein oder Caseinhydrolysat dienen. Beispiele für geeignete Stickstoffquellen sind anorganische Stickstoffquellen wie Nitrate und Ammoniumsalze und organische Stickstoffquellen wie Hefeextrakt, Sojamehl, Baumwollsaatmehl, Casein, Caseinhydrolysat, Weizengluten und Maisquellwasser. Als anorganische Salze können beispielsweise unter anderem Sulfate, Nitrate, Chloride, Carbonate und Phosphate von Natrium, Kalium, Magnesium, Calcium, Zink und Eisen verwendet werden.

Die Kultivierungstemperatur liegt vorzugsweise im Bereich von 10 bis 55 °C, besonders bevorzugt im Bereich von 35 bis 45 °C. Der pH-Wert des Mediums beträgt bevorzugt 3 bis 9, insbesondere 4 bis 8. Die Kultivierung kann entweder in geeigneten Schüttelapparaturen oder in Fermentern mit Mischeinrichtung erfolgen. Bei der Kultivierung ist für eine ausreichende Belüftung Sorge zu tragen. Die Kultivierung kann batchweise, halbkontinuierlich oder kontinuierlich durchgeführt werden. Die Kulturdauer bis zum Erreichen einer maximalen Produktmenge liegt zwischen 4 und 120 Stunden nach dem Animpfen der Kultur. Um die Mikroorganismen vor der toxischen Wirkung der eingesetzten bzw. gebildeten Substanzen zu schützen, kann es vorteilhaft sein, den Kulturmedien Adsorptionsmittel zuzusetzen, z.B. Aktivkohle oder Adsorberharze wie Amberlite XAD-2, Amberlite XAD-7, XAD-16, Lewatit OC 1062 oder OC 1064.

### Vanillylvanillat

Vanillylvanillat stellt den Ester der Vanillinsäure 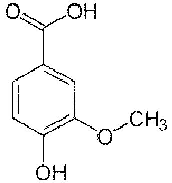 mit Vanillinalkohol dar. Es handelt sich um einen bekannten (CAS 475285-60-0), jedoch wenig eingesetzten Aromastoff, der auch als 4-hydroxy-3-methoxy-(4-hydroxyphenyl)-methylester bezeichnet wird. Als optionaler Parfüm- und Aromastoff wird die Verbindung unter vielen anderen beispielsweise in der WO 2002 090481 A1 oder WO 2002 090479 A1 (P&G) erwähnt.

### STOFFGEMISCHE

Ein besonderer Vorteil der Erfindung besteht darin, dass Synergien zwischen Vanillin und Vanillylvanillat über einen breiten Mischungsbereich festzustellen sind, d.h. in einem Gewichtsverhältnis von etwa 25:75 bis etwa 75:25 und etwa 40:60 bis etwa 60:40 mit einem Schwerpunkt bei etwa gewichtsgleicher Einsatzmenge.

Die Stoffgemische können sich des Weiteren dadurch auszeichnen, dass sie einen Wassergehalt von weniger als etwa 2 und insbesondere von weniger als etwa 1 Gew.-% aufweisen. Vorzugsweise sind die Gemische völlig wasserfrei. Zu diesem Zweck können wässrige und/oder alkoholische Lösungen oder Dispersionen der Stoffgemische getrocknet werden, beispielsweise durch Sprühtrocknung, Wirbelschichttrocknung oder Lyophilisierung (Gefriertrocknung). Die trockenen Zubereitungen können anschließend entweder vermahlen oder granuliert werden.

### VERKAPSELUNG

Die Zubereitungen werden üblicherweise mit Hilfe von Festen Überzugsmaterialien verkapselt, wie beispielsweise Stärken, einschließlich deren Abbauprodukten sowie chemisch oder physikalisch erzeugten Derivaten (insbesondere Dextrine und Maltodextrine), Gelatine, Gummi Arabicum, Agar-Agar, Ghatti Gum, Gellan Gum, modifizierte und nichtmodifizierte Cellulosen, Pullulan, Curdlan, Carrageenane, Alginsäure, Alginate, Pektin, Inulin, Xanthan Gum und Mischungen von zwei oder mehreren dieser Substanzen.

Das feste Verkapselungsmaterial ist vorzugsweise eine Gelatine (insbesondere Schweine-, Rind-, Geflügel- und/oder Fischgelatine), wobei diese vorzugsweise einen Schwellfaktor von größer oder gleich 20, vorzugsweise von größer oder gleich 24 aufweist. Ebenfalls bevorzugt sind Maltodextrine (insbesondere auf Basis von Getreide, speziell Mais, Weizen, Tapioka oder Kartoffeln), die vorzugsweise DE-Werte im Bereich von 10 bis 20 aufweisen. Weiterhin bevorzugt sind Cellulosen (z.B. Celluloseether), Alginate (z.B. Natriumalginat), Carrageenan (z.B. beta-, jota-, lambda- und/oder kappa-Carrageenan), Gummi Arabicum, Curdlan und/oder Agar Agar.

Unter diesen Stoffen ist Gelatine besonders bevorzugt, da sie gut verfügbar ist und mit unterschiedlichen Schwellfaktoren bezogen werden kann. Ganz besonders bevorzugt, insbesondere für orale Anwendungen, sind nahtlose Gelatine- oder Alginatkapseln, deren Hülle sich im Mund oder beim Kauen sehr rasch auflöst oder aufbricht. Entsprechende Kapseln werden beispielsweise in den folgenden Schriften ausführlich EP 0389700 A1**,** US 4,251,195**,** US 6,214,376**,** WO 2003 055587 oder WO 2004 050069 A1**.**

Die Kapseln können alternativ auch Mikrokapseln darstellen. Unter den Begriffen "Mikrokapsel" oder "Nanokapsel" werden vom Fachmann sphärische Aggregate mit einem Durchmesser im Bereich von etwa 0,0001 bis etwa 5 und vorzugsweise 0,005 bis 0,5 mm verstanden, die mindestens einen festen oder flüssigen Kern enthalten, der von mindestens einer kontinuierlichen Hülle umschlossen ist. Genauer gesagt handelt es sich um mit filmbildenden Polymeren umhüllte feindisperse flüssige oder feste Phasen, bei deren Herstellung sich die Polymere nach Emulgierung und Koazervation oder Grenzflächenpolymerisation auf dem einzuhüllenden Material niederschlagen. Nach einem anderen Verfahren werden geschmolzene Wachse in einer Matrix aufgenommen ("microsponge"), die als Mikropartikel zusätzlich mit filmbildenden Polymeren umhüllt sein können. Nach einem dritten Verfahren werden Partikel abwechselnd mit Polyelektrolyten unterschiedlicher Ladung beschichtet ("layer-by-layer"-Verfahren). Die mikroskopisch kleinen Kapseln lassen sich wie Pulver trocknen. Neben einkernigen Mikrokapseln sind auch mehrkernige Aggregate, auch Mikrosphären genannt, bekannt, die zwei oder mehr Kerne im kontinuierlichen Hüllmaterial verteilt enthalten. Ein- oder mehrkernige Mikrokapseln können zudem von einer zusätzlichen zweiten, dritten etc. Hülle umschlossen sein. Die Hülle kann aus natürlichen, halbsynthetischen oder synthetischen Materialien bestehen. Natürlich Hüllmaterialien sind beispielsweise Gummi Arabicum, Agar-Agar, Agarose, Maltodextrine, Alginsäure bzw. ihre Salze, z.B. Natrium- oder Calciumalginat, Fette und Fettsäuren, Cetylalkohol, Collagen, Chitosan, Lecithine, Gelatine, Albumin, Schellack, Polysaccharide, wie Stärke oder Dextran, Polypeptide, Proteinhydrolysate, Sucrose und Wachse. Halbsynthetische Hüllmaterialien sind unter anderem chemisch modifizierte Cellulosen, insbesondere Celluloseester und -ether, z.B. Celluloseacetat, Ethylcellulose, Hydroxypropylcellulose, Hydroxypropylmethylcellulose und Carboxymethylcellulose, sowie Stärkederivate, insbesondere Stärkeether und -ester. Synthetische Hüllmaterialien sind beispielsweise Polymere wie Polyacrylate, Polyamide, Polyvinylalkohol oder Polyvinylpyrrolidon.

Beispiele für Mikrokapseln des Stands der Technik sind folgende Handelsprodukte (in Klammern angegeben ist jeweils das Hüllmaterial) : *Hallcrest Microcapsules* (Gelatine, Gummi Arabicum), *Coletica Thalaspheres* (maritimes Collagen), *Lipotec Millicapseln* (Alginsäure, Agar-Agar), *Induchem Unispheres* (Lactose, mikrokristalline Cellulose, Hydroxypropylmethylcellulose); *Unicerin C30* (Lactose, mikrokristalline Cellulose, Hydroxypropylmethylcellulose), *Kobo Glycospheres* (modifizierte Stärke, Fettsäureester, Phospholipide), *Softspheres* (modifiziertes Agar-Agar) und *Kuhs Probiol Nanospheres* (Phospholipide) sowie *Primaspheres* und *Primasponges* (Chitosan, Alginate) und *Primasys* (Phospholipide).

Chitosanmikrokapseln und Verfahren zu ihrer Herstellung sind aus dem Stand der Technik hinlänglich bekannt **[**WO 01/01926**,** WO 01/01927**,** WO 01/01928**,** WO 01/01929**].** Mikrokapseln mit mittleren Durchmessern im Bereich von 0,0001 bis 5, vorzugsweise 0,001 bis 0,5 und insbesondere 0,005 bis 0,1 mm, bestehend aus einer Hüllmembran und einer die Wirkstoffe enthaltenden Matrix, können beispielsweise erhalten werden, indem man
(a) aus Gelbildnern, kationischen Polymeren und Wirkstoffen eine Matrix zubereitet,
(b) gegebenenfalls die Matrix in einer Ölphase dispergiert,
(c) die dispergierte Matrix mit wässrigen Lösungen anionischer Polymere behandelt und gegebenenfalls dabei die Ölphase entfernt.

Die Schritte (a) und (c) sind dabei insofern austauschbar, als man anstelle der kationischen Polymeren in Schritt (a) anionische Polymere einsetzt und umgekehrt.

Man kann die Kapseln auch erzeugen, indem man den Wirkstoff abwechselnd mit Schichten aus unterschiedlich geladenen Polyelektrolyten einhüllt (layer-by-layer-Technologie). In diesem Zusammenhang sei auf das Europäische Patent EP 1064088 B1 (Max-Planck Gesellschaft) verwiesen.

### ZUBEREITUNGEN FÜR DIE ORALE AUFNAHME

Ein weiterer Gegenstand der Erfindung umfasst Zubereitungen für die orale Aufnahme, welche die erfindungsgemäßen Stoffgemische vorzugsweise in Mengen von etwa 0,01 bis etwa 1 Gew.-%, insbesondere etwa 0,05 bis etwa 0,5 Gew.-% und besonders bevorzugt von etwa 0,8 bis etwa 0,1 Gew.-% enthalten. Bei diesen Zubereitungen kann es sich zum einen um Nahrungsmittel, speziell Backwaren, Getränke, Kaugummis, Süßwaren und dergleichen oder um Mund- und Zahnpflegemittel handeln.

### Nahrungsmittel

Vorzugsweise handelt es sich bei den oralen Zubereitung um Backwaren, beispielsweise Brot, Trockenkekse, Kuchen, sonstiges Gebäck, Süßwaren (beispielsweise Schokoladen, Schokoladenriegelprodukte, sonstige Riegelprodukte, Fruchtgummi, Hart- und Weichkaramellen, Kaugummi), alkoholische oder nicht-alkoholische Getränke (beispielsweise Kaffee, Tee, Eistee, Wein, weinhaltige Getränke, Bier, bierhaltige Getränke, Liköre, Schnäpse, Weinbrände, (carbonisierte) fruchthaltige Limonaden, (carbonisierte) isotonische Getränke, (carbonisierte) Erfrischungsgetränke, Nektare, Schorlen, Obst- und Gemüsesäfte, Frucht- oder Gemüsesaftzubereitungen, Instantgetränke (beispielsweise Instant-Kakao-Getränke, Instant-Tee-Getränke, Instant-Kaffeegetränke, Instant-Fruchtgetränke), Fleischprodukte (beispielsweise Schinken, Frischwurst- oder Rohwurstzubereitungen, gewürzte oder marinierte Frisch- oder Pökelfleischprodukte), Eier oder Eiprodukte (Trockenei, Eiweiß, Eigelb), Getreideprodukte (beispielsweise Frühstückscerealien, Müsliriegel, vorgegarte Fertigreis-Produkte), Milchprodukte (beispielsweise Milchgetränke, Buttermilchgetränke, Milcheis, Joghurt, Kefir, Frischkäse, Weichkäse, Hartkäse, Trockenmilchpulver, Molke, Molkegetränke, Butter, Buttermilch, teilweise oder ganz hydrolisierte Milchprotein-haltige Produkte), Produkte aus Sojaprotein oder anderen Sojabohnen-Fraktionen (beispielsweise Sojamilch und daraus gefertigte Produkte, Fruchtgetränke mit Sojaprotein, Sojalecithin-haltige Zubereitungen, fermentierte Produkte wie Tofu oder Tempe oder daraus gefertigte Produkte), Produkte aus anderen pflanzlichen Proteinquellen, beispielsweise Haferprotein-Getränke, Fruchtzubereitungen (beispielsweise Konfitüren, Fruchteis, Fruchtsoßen, Fruchtfüllungen), Gemüsezubereitungen (beispielsweise Ketchup, Soßen, Trockengemüse, Tiefkühlgemüse, vorgegarte Gemüse, eingekochte Gemüse), Knabberartikel (beispielsweise gebackene oder frittierte Kartoffelchips oder Kartoffelteigprodukte, Extrudate auf Mais- oder Erdnussbasis), Produkte auf Fett- und Ölbasis oder Emulsionen derselben (beispielsweise Mayonnaise, Remoulade, Dressings), sonstige Fertiggerichte und Suppen (beispielsweise Trockensuppen, Instant-Suppen, vorgegarte Suppen), Gewürze, Würzmischungen sowie insbesondere Aufstreuwürzungen (englisch: Seasonings), die beispielsweise im Snackbereich Anwendung finden.

### Nahrungsmittelinhaltsstoffe

Die Nahrungsmittel können weitere Inhaltsstoffe aufweisen, wie z.B. Süßstoffe, Lebensmittelsäuren, Säureregulatoren, Verdickungsmittel und insbesondere Aromastoffe.

### Süßstoffe

Als Süßstoffe oder süß schmeckende Zusatzstoffe kommen zunächst Kohlenhydrate und speziell Zucker in Frage, wie etwa Sucrose/Saccharose, Trehalose, Lactose, Maltose, Melizitose, Raffinose, Palatinose, Lactulose, D-Fructose, D-Glucose, D-Galactose, L-Rhamnose, D-Sorbose, D-Mannose, D-Tagatose, D-Arabinose, L-Arabinose, D-Ribose, D-Glycerinaldehyd, oder Maltodextrin. Ebenfalls geeignet sind pflanzliche Zubereitungen, die diese Stoffe enthalten, beispielsweise auf Basis von Zuckerrüben (Beta vulgaris ssp., Zuckerfraktionen, Zuckersirup, Melasse), Zuckerrohr (Saccharum officinarum ssp., Melasse, Zuckerrohrsirup), Ahornsirup (Acer ssp.) oder Agaven (Agavendicksaft).

In Betracht kommen auch
synthetische, d.h. in der Regel enzymatisch hergestellte Stärke oder Zuckerhydrolysate (Invertzucker, Fructosesirup);
- Fruchtkonzentrate (z.B. auf Basis von Äpfeln oder Birnen);
- Zuckeralkohole (z.B. Erythritol, Threitol, Arabitol, Ribotol, Xylitol, Sorbitol, Mannitol, Dulcitol, Lactitol);
- Proteine (z.B. Miraculin, Monellin, Thaumatin, Curculin, Brazzein);
- Süßstoffe (z.B. Magap, Natriumcyclamat, Acesulfam K, Neohesperidin Dihydrochalcon, Saccharin Natriumsalz, Aspartam, Superaspartam, Neotam, Alitam, Sucralose, Stevioside, Rebaudioside, Lugduname, Carrelame, Sucrononate, Sucrooctate, Monatin, Phenylodulcin);
- Süß schmeckende Aminosäuren (z.B. Glycin, D-Leucin, D-Threonin, D-Asparagin, D-Phenylalanin, D-tryptophn, L-Prolin);
- Weitere süß schmeckende niedermolekulare Substanzen, wie z.B. Hernandulcin, Dihydrochalconglykoside, Glycyrrhizin, Glycerrhetinsäure, ihre Derivate und Salze, Extrakte von Lakritz (Glycyrrhizza glabra ssp.), Lippia dulcis Extrakte, Momordica ssp. Extrakte oder
- Einzelsubstanzen wie z.B. Momordica grosvenori [Luo Han Guo] und die daraus gewonnenen Mogroside, Hydrangea dulcis oder Stevia ssp. (z.B. Stevia rebaudiana) Extrakte.

### Säureregulatoren

Die Nahrungsmittel können Carbonsäuren enthalten. Säuren im Sinne der Erfindung sind bevorzugt in Lebensmitteln zulässige Säuren, insbesondere die hier genannten:
- E 260: - Essigsäure
- E 270: - Milchsäure
- E 290: - Kohlendioxid
- E 296: - Apfelsäure
- E 297: - Fumarsäure
- E 330: - Citronensäure
- E 331: - Natriumcitrat
- E 332: - Kaliumcitrat
- E 333: - Calciumcitrat
- E 334: - Weinsäure
- E 335: - Natriumtartrat
- E 336: - Kaliumtartrat
- E 337: - Natrium-Kaliumtartrat
- E 338: - Phosphorsäure
- E 353: - Metaweinsäure
- E 354: - Calciumtartrat
- E 355: - Adipinsäure
- E 363: - Bernsteinsäure
- E 380: - Triammoniumcitrat
- E 513: - Schwefelsäure
- E 574: - Gluconsäure
- E 575: - Glucono-delta-Lacton

### Säureregulatoren

Säureregulatoren sind Lebensmittelzusatzstoffe, die den Säuregrad oder die Basizität und damit den gewünschten pH-Wert eines Lebensmittels konstant halten. Es handelt sich meist um organische Säuren und deren Salze, Carbonate, seltener auch um anorganische Säuren und deren Salze. Der Zusatz eines Säureregulators verstärkt teils die Stabilität und Festigkeit des Lebensmittels, bewirkt eine erwünschte Ausfällung und verbessert die Wirkung von Konservierungsmitteln. Im Gegensatz zu Säuerungsmitteln werden sie nicht zur Geschmacksveränderung von Lebensmitteln benutzt. Ihre Wirkung beruht auf der Bildung eines Puffersystems im Lebensmittel, bei dem sich auf Zugabe von sauren oder basischen Stoffen der pH-Wert nicht oder nur geringfügig ändert. Beispiele sind:
- E 170: - Calciumcarbonat
- E 260-263: - Essigsäure und Acetate
- E 270: - Milchsäure
- E 296: - Äpfelsäure
- E 297: - Fumarsäure
- E 325-327: - Lactate (Milchsäure)
- E 330-333: - Citronensäure und Citrate
- E 334-337: - Weinsäure und Tartrate
- E 339-341: - Orthophosphate
- E 350-352: - Malate (Äpfelsäure)
- E 450-452: - Di-, Tri- und Polyphosphate
- E 500-504: - Carbonate (Kohlensäure)
- E 507: - Salzsäure und ChlorideE 513-517Schwefelsäure und Sulfate
- E 524-528: - Hydroxide
- E 529-530: - Oxide
- E 355-357: - Adipinsäure und Adipate
- E 574-578: - Gluconsäure und Gluconate

### Verdickungsmittel

Verdickungsmittel sind Stoffe, die in erster Linie in der Lage sind, Wasser zu binden. Durch Entzug von ungebundenem Wasser kommt es zur Erhöhung der Viskosität. Ab einer für jedes Verdickungsmittel charakteristischen Konzentration treten zu diesem Effekt auch noch Netzwerkeffekte auf, die zu einer meist überproportionalen Erhöhung der Viskosität führen. Man spricht in diesem Fall davon, dass Moleküle miteinander 'kommunizieren', d.h. verschlaufen. Bei den meisten Verdickungsmitteln handelt es sich um lineare oder verzweigte Makromoleküle (z. B. Polysaccharide oder Proteine), die durch intermolekulare Wechselwirkungen, wie Wasserstoffbrücken, hydrophobe Wechselwirkungen oder Ionenbeziehungen miteinander interagieren können. Extremfälle von Dickungsmitteln sind Schichtsilikate (Bentonite, Hectorite) oder hydratisierte SiO₂-Partikel, die als Teilchen dispergiert vorliegen und in ihrer festkörperartigen Struktur Wasser binden können bzw. aufgrund der beschriebenen Wechselwirkungen miteinander interagieren können. Beispiele sind:
- E 400: - Alginsäure
- E 401: - Natriumalginat
- E 402: - Kaliumalginat
- E 403: - Ammoniumalginat
- E 404: - Calciumalginat
- E 405: - Propylenglycolalginat
- E 406: - Agar Agar
- E 407: - Carrgeen, Furcelleran
- E 407: - Johannisbrotkernmehl
- E 412: - Guarkernmehl
- E 413: - Traganth
- E 414: - Gummi arabicum
- E 415: - Xanthan
- E 416: - Karaya (Indischer Traganth)
- E 417: - Tarakernmehl (Peruanisches Johannisbrotkernmehl)
- E 418: - Gellan
- E 440: - Pektin, Opekta
- E 440ii: - Amidiertes Pektin
- E 460: - Mikrokristalline Cellulose, Cellulosepulver
- E 461: - Methylcellulose
- E 462: - Ethylcellulose
- E 463: - Hydroxypropylcellulose
- E 465: - Methylethylcellulose
- E 466: - Carboxymethylcellulose, Natriumcarboxymethylcellulose

### Aromastoffe

Die Erfindung erlaubt insbesondere auch den Einsatz von Aromastoffen mit Ester-, Aldehyd- oder Lactonstruktur, die in Gegenwart von Titandioxid und unter Lichteinfluss besonders schnell abgebaut werden. Die Erfindung sorgt somit auch für eine verbesserte Stabilität, speziell Lagerstabilität der Aromastoffe.

Die erfindungsgemäßen oralen Zubereitungen können einen oder mehrere Aromastoffe enthalten. Typische Beispiele umfassen: Acetophenon, Allylcapronat, alpha-Ionon, beta-Ionon, Anisaldehyd, Anisylacetat, Anisylformiat, Benzaldehyd, Benzothiazol, Benzylacetat, Benzylalkohol, Benzylbenzoat, beta-Ionon, Butylbutyrat, Butylcapronat, Butylidenphthalid, Carvon, Camphen, Caryophyllen, Cineol, Cinnamylacetat, Citral, Citronellol, Citronellal, Citronellylacetat, Cyclohexylacetat, Cymol, Damascon, Decalacton, Dihydrocumarin, Dimethylanthranilat, Dimethylanthranilat, Dodecalacton, Ethoxyethylacetat, Ethylbuttersäure, Ethylbutyrat, Ethylcaprinat, Ethylcapronat, Ethylcrotonat, Ethylfuraneol, Ethylguajakol, Ethylisobutyrat, Ethylisovalerianat, Ethyllactat, Ethylmethylbutyrat, Ethylpropionat, Eucalyptol, Eugenol, Ethylheptylat, 4-(p-Hydroxyphenyl)-2-butanon, gamma-Decalacton, Geraniol, Geranylacetat, Geranylacetat, Grapefruitaldehyd, Methyldihydrojasmonat (z.B. Hedion®), Heliotropin, 2-Heptanon, 3-Heptanon, 4-Heptanon, trans-2-Heptenal, cis-4-Heptenal, trans-2-Hexenal, cis-3-Hexenol, trans-2-Hexensäure, trans-3-Hexensäure, cis-2-Hexenylacetat, cis-3-Hexenylacetat, cis-3-Hexenylcapronat, trans-2-Hexenylcapronat, cis-3-Hexenylformiat, cis-2-Hexylacetat, cis-3-Hexylacetat, trans-2-Hexylacetat, cis-3-Hexylformiat, para-Hydroxybenzylaceton, Isoamylalkohol, Isoamylisovalerianat, Isobutylbutyrat, Isobutyraldehyd, Isoeugenolmethylether, Isopropylmethylthiazol, Laurinsäure, Leavulinsäure, Linalool, Linalooloxid, Linalylacetat, Menthol, Menthofuran, Methylanthranilat, Methylbutanol, Methylbuttersäure, 2-Methylbutylacetat, Methylcapronat, Methylcinnamat, 5-Methylfurfural, 3,2,2-Methylcyclopentenolon, 6,5,2-Methylheptenon, Methyldihydrojasmonat, Methyljasmonat, 2-Methylmethylbutyrat, 2-Methyl-2-Pentenolsäure, Methylthiobutyrat, 3,1-Methylthiohexanol, 3-Methylthiohexylacetat, Nerol, Nerylacetat, trans,trans-2,4-Nonadienal, 2,4-Nonadienol, 2,6-Nonadienol, 2,4-Nonadienol, Nootkaton, delta Octalacton, gamma Octalacton, 2-Octanol, 3-Octanol, 1,3-Octenol, 1-Octylacetat, 3-Octylacetat, Palmitinsäure, Paraldehyd, Phellandren, Pentandion, Phenylethylacetat, Phenylethylalkohol, Phenylethylalkohol, Phenylethylisovalerianat, Piperonal, Propionaldehyd, Propylbutyrat, Pulegon, Pulegol, Sinensal, Sulfurol, Terpinen, Terpineol, Terpinolen, 8,3-Thiomenthanon, 4,4,2-Thiomethylpentanon, Thymol, delta-Undecalacton, gamma-Undecalacton, Valencen, Valeriansäure, Vanillin, Acetoin, Ethylvanillin, Ethylvanillinisobutyrat (= 3-Ethoxy-4-isobutyryloxybenzaldehyd), 2,5-Dimethyl-4-hydroxy-3(2H)-furanon und dessen Abkömmlinge (dabei vorzugsweise Homofuraneol (= 2-Ethyl-4-hydroxy-5-methyl-3(2H)-furanon), Homofuronol (= 2-Ethyl-5-methyl-4-hydroxy-3(2H)-furanon und 5-Ethyl-2-methyl-4-hydroxy-3(2H)-furanon), Maltol und Maltol-Abkömmlinge (dabei vorzugsweise Ethylmaltol), Cumarin und Cumarin-Abkömmlinge, gamma-Lactone (dabei vorzugsweise gamma-Undecalacton, gamma-Nonalacton, gamma-Decalacton), delta-Lactone (dabei vorzugsweise 4-Methyldeltadecalacton, Massoilacton, Deltadecalacton, Tuberolacton), Methylsorbat, Divanillin, 4-Hydroxy-2(oder 5)-ethyl-5(oder 2)-methyl-3(2H)furanon, 2-Hydroxy-3-methyl-2-cyclopentenon, 3-Hydroxy-4,5-dimethyl-2(5H)-furanon, Essigsäureisoamylester, Buttersäureethylester, Buttersäure-n-butylester, Buttersäureisoamylester, 3-Methylbuttersäureethylester, n-Hexansäureethylester, n-Hexansäureallylester, n-Hexansäure-n-butylester, n-Octansäureethylester, Ethyl-3-methyl-3-phenylglycidat, Ethyl-2-trans-4-cis-decadienoat, 4-(p-Hydroxyphenyl)-2-butanon, 1,1-Dimethoxy-2,2,5-trimethyl-4-hexan, 2,6-Dimethyl-5-hepten-1-al und Phenylacetaldehyd, 2-Methyl-3-(methylthio)furan, 2-Methyl-3-furanthiol, bis(2-Methyl-3-furyl)disulfid, Furfurylmercaptan, Methional, 2-Acetyl-2-thiazolin, 3-Mercapto-2-pentanon, 2,5-Dimethyl-3-furanthiol, 2,4,5-Trimethylthiazol, 2-Acetylthiazol, 2,4-Dimethyl-5-ethylthiazol, 2-Acetyl-1-pyrrolin, 2-Methyl-3-ethylpyrazin, 2-Ethyl-3,5-dimethylpyrazin, 2-Ethyl-3,6-dimethylpyrazin, 2,3-Diethyl-5-methylpyrazin, 3-Isopropyl-2-methoxypyrazin, 3-Isobutyl-2-methoxypyrazin, 2-Acetylpyrazin, 2-Pentylpyridin, (E,E)-2,4-Decadienal, (E,E)-2,4-Nonadienal, (E)-2-Octenal, (E)-2-Nonenal, 2-Undecenal, 12-Methyltridecanal, 1-Penten-3-on, 4-Hydroxy-2,5-dimethyl-3(2H)-furanon, Guajakol, 3-Hydroxy-4,5-dimethyl-2(5H)-furanon, 3-Hydroxy-4-methyl-5-ethyl-2(5H)-furanon, Zimtaldehyd, Zimtalkohol, Methylsalicylat, Isopulegol sowie (hier nicht explizit genannte) Stereoisomere, Enantiomere, Stellungsisomere, Diastereomere, cis/trans-Isomere bzw. Epimere dieser Substanzen.

### Kaugummis

Bei den bevorzugten oralen Zubereitungen kann es sich auch um Kaugummis handeln. Diese Produkte enthalten typischerweise eine wasserunlösliche und eine wasserlösliche Komponente.

Die wasserunlösliche Basis, die auch als "Gummibasis" bezeichnet wird, umfasst üblicherweise natürliche oder synthetische Elastomere, Harze, Fette und Öle, Weichmacher, Füllstoffe, Farbstoffe sowie gegebenenfalls Wachse. Der Anteil der Basis an der Gesamtzusammensetzung macht üblicherweise 5 bis 95, vorzugsweise 10 bis 50 und insbesondere 20 bis 35 Gew.-% aus. Typischerweise setzt sich die Basis aus 20 bis 60 Gew.-% synthetischen Elastomeren, 0 bis 30 Gew.-% natürlichen Elastomeren, 5 bis 55 Gew.-% Weichmachern, 4 bis 35 Gew.-% Füllstoffe und in untergeordneten Mengen Zusatzstoffe wie Farbstoffe, Antioxidantien und dergleichen zusammen, mit der Maßgabe, dass sie allenfalls in geringen Mengen wasserlöslich sind.

Als geeignete synthetische Elastomere kommen beispielsweise Polyisobutylene mit durchschnittlichen Molekulargewichten (nach GPC) von 10.000 bis 100.000 und vorzugsweise 50.000 bis 80.000, Isobutylen-Isopren-Copolymere ("Butyl Elastomere"), Styrol-Butadien-Copolymere (Styrol:Butadien-Verhältnis z.B. 1: 3 bis 3: 1), Polyvinylacetate mit durchschnittlichen Molekulargewichten (nach GPC) von 2.000 bis 90.000 und vorzugsweise 10.000 bis 65.000, Polyisoprene, Polyethylen, Vinylacetat-Vinyllaurat-Copolymere und deren Gemische. Beispiele für geeignete natürliche Elastomere sind Kautschuks wie etwa geräucherter oder flüssiger Latex oder Guayule sowie natürliche Gummistoffe wie Jelutong, Lechi caspi, Perillo, Sorva, Massaranduba balata, Massaranduba chocolate, Nispero, Rosindinba, Chicle, Gutta hang 1kang sowie deren Gemische. Die Auswahl der synthetischen und natürlichen Elastomere und deren Mischungsverhältnisse richtet sich im Wesentlichen danach, ob mit den Kaugummis Blasen erzeugt werden sollen ("bubble gums") oder nicht. Vorzugsweise werden Elastomergemische eingesetzt, die Jelutong, Chicle, Sorva und Massaranduba enthalten.

In den meisten Fällen erweisen sich die Elastomere in der Verarbeitung als zu hart oder zu wenig verformbar, so dass es sich als vorteilhaft erwiesen hat, spezielle Weichmacher mitzuverwenden, die natürlich insbesondere auch alle Anforderungen an die Zulassung als Nahrungsmittelzusatzstoffe erfüllen müssen. In dieser Hinsicht kommen vor allem Ester von Harzsäuren in Betracht, beispielsweise Ester von niederen aliphatischen Alkoholen oder Polyolen mit ganz oder teilweise gehärteten, monomeren oder oligomeren Harzsäuren. Insbesondere werden für diesen Zweck die Methyl-, Glycerin-, oder Pentareythritester sowie deren Gemische eingesetzt. Alternativ kommen auch Terpenharze in Betracht, die sich von alpha-Pinen, beta-Pinen, delta-Limonen oder deren Gemischen ableiten können.

Als Füllstoffe oder Texturiermittel kommen Magnesium- oder Calciumcarbonat, gemahlener Bimsstein, Silicate, speziell Magnesium- oder Aluminiumsilicate, Tone, Aluminiumoxide, Talkum, Titandioxid, Mono-, Di- und Tricalciumphosphat sowie Cellulosepolymere in Frage.

Geeignete Emulgatoren sind Talg, gehärteter Talg, gehärtete oder teilweise gehärtete pflanzliche Öle, Kakaobutter, Partialglyceride, Lecithin, Triacetin und gesättigte oder ungesättigte Fettsäuren mit 6 bis 22 und vorzugsweise 12 bis 18 Kohlenstoffatomen sowie deren Gemische.

Als Farbstoffe und Weißungsmittel kommen beispielsweise die für die Färbung von Lebensmitteln zugelassenen FD und C-Typen, Pflanzen- und Fruchtextrakte sowie Titandioxid in Frage.

Die Basismassen können Wachse enthalten oder wachsfrei sein; Beispiele für wachsfreie Zusammensetzungen finden sich unter anderem in der Patentschrift US 5,286,500**,** auf deren Inhalt hiermit ausdrücklich Bezug genommen wird.

Zusätzlich zu der wasserunlöslichen Gummibasis enthalten Kaugummizubereitungen regelmäßig einen wasserlösliche Anteil, der beispielsweise von Softener, Süßstoffen, Füllstoffen, Geschmacksstoffen, Geschmacksverstärkern, Emulgatoren, Farbstoffen, Säuerungsmitteln, Antioxidantien und dergleichen gebildet werden, hier mit der Maßgabe, dass die Bestandteile eine wenigstens hinreichende Wasserlöslichkeit besitzen. In Abhängigkeit der Wasserlöslichkeit der speziellen Vertreter können demnach einzelne Bestandteile sowohl der wasserunlöslichen wie auch der wasserlöslichen Phase angehören. Es ist jedoch auch möglich, Kombinationen beispielsweise eines wasserlöslichen und eines wasserunlöslichen Emulgators einzusetzen, wobei sich die einzelnen Vertreter, dann in unterschiedlichen Phasen befinden. Üblicherweise macht der wasserunlösliche Anteil 5 bis 95 und vorzugsweise 20 bis 80 Gew.-% der Zubereitung aus.

Wasserlösliche Softener oder Plastifiziermittel werden den Kaugummizusammensetzungen hinzugegeben um die Kaubarkeit und das Kaugefühl zu verbessern und sind in den Mischungen typischerweise in Mengen von 0,5 bis 15 Gew.-% zugegen. Typische Beispiele sind Glycerin, Lecithin sowie wässrige Lösungen von Sorbitol, gehärteten Stärkehydrolysaten oder Kornsirup.

Als Süßstoffe kommen sowohl zuckerhaltige wie zuckerfreie Verbindungen in Frage, die in Mengen von 5 bis 95, vorzugsweise 20 bis 80 und insbesondere 30 bis 60 Gew.-% bezogen auf die Kaugummizusammensetzung eingesetzt werden. Typische Saccharid-Süssstoffe sind Sucrose, Dextrose, Maltose, Dextrin, getrockneter Invertzucker, Fructose, Levulose, Galactose, Kornsirup sowie deren Gemische. Als Zuckerersatzstoffe kommen Sorbitol, Mannitol, Xylitol, gehärtete Stärkehydrolysate, Maltitol und deren Gemische in Frage. Weiterhin kommen als Zusatzstoffe auch sogenannte HIAS ("High Intensity Articifical Sweeteners") in Betracht, wie beispielsweise Sucralose, Aspartam, Acesulfamsalze, Alitam, Saccharin und Saccharinsalze, Cyclamsäure und deren Salze, Glycyrrhizine, Dihydrochalcone, Thaumatin, Monellin und dergleichen alleine oder in Abmischungen. Besonders wirksam sind auch die hydrophoben HIAS, die Gegenstand der internationalen Patentanmeldung WO 2002 091849 A1 (Wrigleys) sowie Stevia Extrakte und deren aktiven Bestandteile, insbesondere Ribeaudiosid A sind. Die Einsatzmenge dieser Stoffe hängt in erster Linie von ihrem Leistungsvermögen ab und liegt typischerweise im Bereich von 0,02 bis 8 Gew.-%.

Insbesondere für die Herstellung kalorienarmer Kaugummis eignen sich Füllstoffe wie beispielsweise Polydextrose, Raftilose, Rafitilin, Fructooligosaccharide (NutraFlora), Palatinoseoligosaaccharide, Guar Gum Hydrolysate (Sun Fiber) sowie Dextrine.

Die Auswahl an weiteren Geschmacksstoffen ist praktisch unbegrenzt und für das Wesen der Erfindung unkritisch. Üblicherweise liegt der Gesamtanteil aller Geschmacksstoffe bei 0,1 bis 15 und vorzugsweise 0,2 bis 5 gew.-% bezogen auf die Kaugummizusammensetzung. Geeignete weitere Geschmacksstoffe stellen beispielsweise essentielle Öle, synthetische Aromen und dergleichen dar, wie etwa Anisöl, Sternanisöl, Kümmelöl, Eukalyptusöl, Fenchelöl, Citronenöl, Wintergrünöl, Nelkenöl, und dergleichen, wie sie auch beispielsweise in Mund- und Zahnpflegemittel Verwendung finden.

Die Kaugummis können des weiteren Hilfs- und Zusatzstoffe enthalten, die beispielsweise für die Zahnpflege, speziell zur Bekämpfung von Plaque und Gingivitis geeignet sind, wie z.B. Chlorhexidin, CPC oder Trichlosan. Weiter können pH-Regulatoren (z.B. Puffer oder Harnstoff), Wirkstoffe gegen Karies (z.B. Phosphate oder Fluoride), biogene Wirkstoffe (Antikörper, Enzyme, Koffein, Pflanzenextrakte) enthalten sein, solange diese Stoffe für Nahrungsmittel zugelassen sind und nicht in unerwünschter Weise miteinander in Wechselwirkung treten.

### Mund- und Zahnpflegemittel

Erfindungsgemäße oral konsumierbare süß schmeckende Produkte können auch der Mund- und Zahnreinigung und-pflege dienen. Beispiele hierfür sind Zahnpasten, Zahngele, Zahnpulver, Mundwässer und dergleichen. Unter Zahnpasten oder Zahncremes werden im allgemeinen gelförmige oder pastöse Zubereitungen aus Wasser, Verdickungsmitteln, Feuchthaltemitteln, Schleif- oder Putzkörpern, Tensiden, Süßmitteln, Aromastoffen, deodorierenden Wirkstoffen sowie Wirkstoffen gegen Mund- und Zahnerkrankungen verstanden. In die erfindungsgemäßen Zahnpasten können alle üblichen Putzkörper, wie z. B. Kreide, Dicalciumphosphat, unlösliches Natriummetaphosphat, Aluminiumsilikate, Calciumpyrophosphat, feinteilige Kunstharze, Kieselsäuren, Aluminiumoxid und Aluminiumoxidtrihydrat eingesetzt werden.

Bevorzugt geeignete Putzkörper für die erfindungsgemäßen Zahnpasten sind vor allem feinteilige Xerogelkieselsäuren, Hydrogelkieselsäuren, Fällungskieselsäuren, Aluminiumoxid-trihydrat und feinteiliges alpha -Aluminiumoxid oder Mischungen dieser Putzkörper in Mengen von 15 bis 40 Gew.-% der Zahnpasta. Als Feuchthaltemittel kommen vorwiegend niedermolekulare Polyethylenglykole, Glycerin, Sorbit oder Mischungen dieser Produkte in Mengen bis zu 50 Gew.-% in Frage. Unter den bekannten Verdickungsmitteln sind die verdickenden, feinteiligen Gelkieselsäuren und Hydrokolloide, wie z. B. Carboxymethylcellulose, Hydroxyethylcellulose, Hydroxypropylguar, Hydroxyethylstärke, Polyvinylpyrrolidon, hochmolekulares Polyethylenglykol, Pflanzengummen wie Traganth, Agar-Agar, Carragheenmoos, Gummi arabicum, Xantham-Gum und Carboxyvinylpolymere (z. B. Carbopol®-Typen) geeignet. Zusätzlich zu den Mischungen aus menthofuran und Mentholverbindungen können die Mund- und Zahnpflegemittel insbesondere oberflächenaktive Stoffe, bevorzugt anionische und nichtionische schaumstarke Tenside, wie die bereits oben genannten Stoffe, insbesondere aber Alkylethersulfat-Salze, Alkylpolyglucoside und deren Gemische.

Weitere übliche Zahnpastenzusätze sind:
- Konservierungsmittel und antimikrobielle Stoffe wie z. B. p- Hydroxybenzösäuremethyl-, -ethyl- oder -propylester, Natriumsorbat, Natriumbenzoat, Bromchlorophen, Phenylsalicylsäureester, Thymol und dergleichen;
- Antizahnsteinwirkstoffe, z. B. Organophosphate wie 1-Hydroxyethan- 1.1-diphosphonsäure, 1-Phosphonpropan-1,2,3-tricarbonsäure und andere, die z. B. aus US 3,488,419**,** DE 2224430 A1 und DE 2343196 A1 bekannt sind;
- andere karieshemmende Stoffe wie z. B. Natriumfluorid, Natriummonofluorphosphat, Zinnfluorid;
- Süssungsmittel, wie z. B. Saccharin-Natrium, Natrium-Cyclamat, Sucrose, Lactose, Maltose, Fructose oder Apartam®, (L-Aspartyl- L-phenylalanin-methylester), Stiviaextrakte oder deren süßenden Bestandteile, insbesondere Ribeaudioside;
- Zusätzliche Aromen wie z. B. Eukalyptusöl, Anisöl, Fenchelöl, Kümmelöl, Methylacetat, Zimtaldehyd, Anethol, Vanillin, Thymol sowie Mischungen dieser und anderer natürlicher und synthetischer Aromen;
- Pigmente wie z. B. Titandioxid;
- Farbstoffe;
- Puffersubstanzen wie z. B. primäre, sekundäre oder tertiäre Alkaliphosphate oder Citronensäure/Natriumcitrat;

wundheilende und entzündungshemmende Stoffe wie z. B. Allantoin, Harnstoff, Azulen, Kamillenwirkstoffe und Acetylsalicylsäurederivate.

Eine bevorzugte Ausführung der oralen Zubereitungen sind Zahnpasten in Form einer wässrigen, pastösen Dispersion, enthaltend Poliermittel, Feuchthaltemittel, Viskositätsregulatoren und gegebenenfalls weitere übliche Komponenten, sowie die Mischung aus Menthofuran und Mentholverbindungen in Mengen von 0,5 bis 2 Gew.-% enthalten.

In Mundwässern ist eine Kombination mit wässrig-alkoholischen Lösungen verschiedener Grädigkeit von ätherischen Ölen, Emulgatoren, adstringierenden und tonisierenden Drogenauszügen, zahnsteinhemmenden, antibakteriellen Zusätzen und Geschmackskorrigentien ohne weiteres möglich. Eine weitere bevorzugte Ausführung der Erfindung ist ein Mundwasser in Form einer wässrigen oder wässrig-alkoholischen Lösung enthaltend die Mischung aus Menthofuran und Mentholverbindungen in Mengen von 0,5 bis 2 Gew.-%. In Mundwässern, die vor der Anwendung verdünnt werden, können mit, entsprechend dem vorgesehenen Verdünnungsverhältnis, höheren Konzentrationen ausreichende Effekte erzielt werden.

Zur Verbesserung des Fließverhaltens können ferner Hydrotrope, wie beispielsweise Ethanol, Isopropylalkohol, oder Polyole eingesetzt werden; diese Stoffe entsprechen weitgehend den eingangs geschildern Trägern. Polyole, die hier in Betracht kommen, besitzen vorzugsweise 2 bis 15 Kohlenstoffatome und mindestens zwei Hydroxylgruppen. Die Polyole können noch weitere funktionelle Gruppen, insbesondere Aminogruppen, enthalten bzw. mit Stickstoff modifiziert sein. Typische Beispiele sind
- Glycerin;
- Alkylenglycole, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton;
- technische Oligoglyceringemische mit einem Eigenkondensationsgrad von 1,5 bis 10 wie etwa technische Diglyceringemische mit einem Diglyceringehalt von 40 bis 50 Gew.-%;
- Methyolverbindungen, wie insbesondere Trimethylolethan, Trimethylolpropan, Trimethylolbutan, Pentaerythrit und Dipentaerythrit;
- Niedrigalkylglucoside, insbesondere solche mit 1 bis 8 Kohlenstoffen im Alkylrest, wie beispielsweise Methyl- und Butylglucosid;
- Zuckeralkohole mit 5 bis 12 Kohlenstoffatomen, wie beispielsweise Sorbit oder Mannit,
- Zucker mit 5 bis 12 Kohlenstoffatomen, wie beispielsweise Glucose oder Saccharose;
- Aminozucker, wie beispielsweise Glucamin;
- Dialkoholamine, wie Diethanolamin oder 2-Amino-1,3-propandiol.

Als Konservierungsmittel eignen sich beispielsweise Phenoxyethanol, Formaldehydlösung, Parabene, Pentandiol oder Sorbinsäure sowie die unter der Bezeichnung Surfacine® bekannten Silberkomplexe und die in Anlage 6, Teil A und B der Kosmetikverordnung aufgeführten weiteren Stoffklassen.

Als Parfümöle seien genannt Gemische aus natürlichen und synthetischen Riechstoffen. Natürliche Riechstoffe sind Extrakte von Blüten (Lilie, Lavendel, Rosen, Jasmin, Neroli, Ylang-Ylang), Stengeln und Blättern (Geranium, Patchouli, Petitgrain), Früchten (Anis, Koriander, Kümmel, Wacholder), Fruchtschalen (Bergamotte, Zitrone, Orangen), Wurzeln (Macis, Angelica, Sellerie, Kardamon, Costus, Iris, Calmus), Hölzern (Pinien-, Sandel-, Guajak-, Zedern-, Rosenholz), Kräutern und Gräsern (Estragon, Lemongras, Salbei, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethylmethylphenylglycinat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone, α-Isomethylionon und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labolanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romilllat, Irotyl und Floramat allein oder in Mischungen, eingesetzt.

Als Aromen kommen beispielsweise Pfefferminzöl, Krauseminzöl, Anisöl, Sternanisöl, Kümmelöl, Eukalyptusöl, Fenchelöl, Citronenöl, Wintergrünöl, Nelkenöl, Menthol und dergleichen in Frage.

### GEWERBLICHE ANWENDBARKEIT

Ein weiterer Gegenstand der Erfindung betrifft die Verwendung der erfindungsgemäßen Stoffgemische als Aromastoffe, vorzugsweise in Mengen von etwa 0,01 bis etwa 1 Gew.-%, insbesondere etwa 0,05 bis 0,5 Gew.-% - jeweils bezogen auf die konfektionierten Endprodukte.

### BEISPIELE

### HERSTELLBEISPIEL H1: Vanillin ex Maiskleie

100 g dunkelgefärbter Maiskleie wurden in einen 500 ml Dreihalskolben gegeben und mit 5 g einer auf einem Träger immobilisierten Ferulasäureesterase, 20 ml Wasser und 20 ml Isopropylalkohol versetzt. Die Mischung wurde etwa 8 h bei 32 °C gerührt und dann mit 100 ml Hexan versetzt, um alle unpolaren Anteile in Lösung zu bringen. Die organische Phase wurde abgetrennt, die wässrige Lösung mit verdünnter Schwefelsäure versetzt und ein pH-Wert von etwa 2 eingestellt, bei dem die Ferulasäure ausfiel. Es konnten 5 g Ferulasäure mit einer Reinheit von etwa 80 % abfiltriert werden. Diese Menge wurde in heißem Wasser gelöst und durch erneutes Abkühlen umkristallisiert. Es wurden 3,5 g Ferulasäure mit einer Reinheit von 98 % erhalten.

Ein 500 ml Erlenmeyerkolben mit seitlichem Einstich wurde mit 100 Medium, bestehend aus 1 g Malzextrakt, 0,4 g Glucose und 0,4 g Hefeextrakt ad 100 mit Wasser aufgefüllt und anschließend 20 min bei 121 °C sterilisiert. Nach dem Abkühlen wurde der Kolben mit 200 µl einer eingefrorenen Glycerinkultur *Amycolatopsis sp.* DSM 9991 beimpft. Die Kultur wurde auf einer rotierenden Schüttelmaschine bei 45 °C und 100 Upm inkubiert. Nach 24 h wurde die Kultur zur Beimpfung des Produktionsmediums verwendet.

Acht 500 ml Erlenmeyerkolben mit seitlichem Einstich wurden mit je 100 ml Medium (4 g/l Glucose, 10 g/l Malzextrakt) gefüllt und anschließend 20 min bei 121 °C sterilisiert. Nach dem Abkühlen wurden die Kolben mit je 2 ml einer Kultur von *Amycolatopsis sp.* DSM 9991 (wie oben beschrieben) angeimpft. Die Kulturen wurden auf einer rotierenden Schüttelmaschine bei 37 °C und 100 Upm inkubiert. 16 h nach dem Animpfen wurden 20 ml, nach 24 h 409 ml und nach 44 h nochmals 10 ml einer sterilisierten Ferulasäurelösung aus Teil A des Beispiels zu jedem Kolben zugegeben. Nach 46 h wurde die Kulturbrühe der Kolben vereinigt und der Gehalt an Vanillin und nicht umgesetzter Ferulasäure bestimmt: Vanillin: 93,6 %; nicht umgesetzte Ferulasäure: 1.250 ppm.

### ANWENDUNGSBEISPIELE 1 bis 3, VERGLEICHBEISPIELE V1 und V2

### Sensorische Beurteilung

Natürliches Vanillin (C), Vanillin aus Ferulasäure ex Maiskleie, drei synthetische Mischungen dieser Vanillequalität mit im Handel erhältlichen Vanillylvanillat sowie Vanillylvanillat alleine wurden hinsichtlich ihrer Ähnlichkeit zu natürlicher Vanille sensorisch beurteilt. Die Beurteilung erfolgte durch ein Panel von 5 geschulten Personen. Zu Grunde gelegt wurde eine Matrix bestehend aus einer 5 Gew.-%igen Zuckerlösung und eine Einsatzkonzentration von 30 ppm. Neben der subjektiven Geschmacksbeschreibung wurde die Ähnlichkeit der Aromen im Vergleich zu natürlichem Vanillin aus Vanilleschoten jeweils auf einer Skala von 1 (sehr unähnlich) bis 10 (praktisch identisch) beschrieben. Die Ergebnisse sind in **Tabelle 1** zusammengefasst; die sensorische Beurteilung stellt den Mittelwert der Testergebnisse dar. Die Beispiele 1 bis 3 sind erfinderisch, die Beispiele V1 und V2 dienen zum Vergleich.

**Tabelle 1**

| Zusammensetzung und Geschmackseindruck; Mengenangaben als Gew.-% | | | | | | |
|---|---|---|---|---|---|---|
| | **C** | **V1** | **1** | **2** | **3** | **V2** |
| ***Zusammensetzung*** | | | | | | |
| Vanillin, natürlich | 100 | 0 | 0 | 0 | 0 | 0 |
| Vanillin, synthetisch¹ | 0 | 100 | 25 | 50 | 75 | 0 |
| Vanillylvanillat | 0 | 0 | 75 | 50 | 25 | 100 |

| ***Sensorische Beurteilung*** | | | | | | |
|---|---|---|---|---|---|---|
| Süß | 8 | 6 | 7 | 8 | 7 | 4 |
| Balsamisch | 6 | 4 | 3 | 3 | 2 | 2 |
| Phenolisch | 3 | 3 | 3 | 2 | 2 | 2 |
| Ähnlichkeit | 10 | 6 | 7 | 8 | 7 | 4 |

Die Beispiele und Vergleichsbeispiele zeigen bezüglich der sensorischen Beurteilung ein synergistisches Verhalten von Vanillin und Vanillylvanillat gegenüber den einzelnen Komponenten. Die Mischungen erweisen sich sowohl bezüglich der einzelnen Geschmackskomponenten auch in der Gesamtnote dem natürlichen Vanillin wesentlich ähnlicher als die Einzelsubstanzen.

### FORMULIERUNGSBEISPIELE

**Tabelle 2a**

| Kaugummimassen | | | | | | | | |
|---|---|---|---|---|---|---|---|---|
| **Zusammensetzung** | **A** | **B** | **c** | **D** | **E** | **F** | **G** | **H** |
| Polyisobutylen (MW 20.000) | 30,0 | 30,0 | 30,0 | 40,0 | 20.0 | 20.0 | 25.0 | 30.0 |
| Glucose | 51,0 | 51,0 | 51,0 | 42,5 | - | - | - | - |
| Kornsirup | 10,0 | 10,0 | 10,0 | 8,0 | - | - | - | - |
| Sorbitol | - | - | - | - | 51,0 | 51,0 | 47,5 | 44,5 |
| Mannitol | - | - | - | - | 5,0 | 5,0 | 4,3 | 3,6 |
| Glycerin | 1,8 | 1,8 | 1,8 | 1,8 | 8,0 | 8,0 | 8,0 | 7,0 |
| Lycasin:Glycerin (1:1) | - | - | - | - | 8,2 | 8,2 | 8,0 | 7,0 |
| Lecithin | - | - | - | - | 0,2 | 0,2 | 0,2 | 0,2 |
| Vanillin/Vanillylvanillat | 0.5 | 0.3 | 0.1 | 0.2 | 0.1 | 0.5 | 0.3 | 0.2 |
| Wasser | Ad 100 | | | | | | | |

**Tabelle 2b**

| Zusammensetzung Zahnpaste | | |
|---|---|---|
| **Komponente** | **Handelsprodukt** | **Menge [Gew.-%]** |
| Fällungskieselsäure | Sident® 12 DS | 18,0 |
| Verdickungskieselsäure | Aerosil® 200 | 0,8 |
| Sorbit | | 17,5 |
| Glycerin | | 17,5 |
| Carboxymethylcellulose | Relatin® 100 SR | 0,9 |
| Natriumlaurylsulfat | Texapon® K1296 | 2,0 |
| Natriumfluorid | | 0,22 |
| Saccharin-Natrium | | 0,2 |
| Vanillin/Vanillylvanillat | | 0.2 |
| Wasser | | Ad 100 |

**Tabelle 2c**

| Zusammensetzung Mundwasser | | |
|---|---|---|
| **Komponente** | **Handelsprodukt** | **Menge [Gew.-%]** |
| Ethanol (96%ig) | | 10,0 |
| Sorbitanmonolaurat+20EO | Tween® 20 | 0,4 |
| Vanillin/Vanillylvanillat | | 0,3 |
| Sorbit (70%ige wässrige Lösung) | | 8,0 |
| p-Hydroxybenzoesäuremethylester | | 0,2 |
| Wasser | | Ad 100 |

**Tabelle 2d**

| Zuckerfreies Bonbon | | | |
|---|---|---|---|
| **Komponente** | **X** | **XI** | **XII** |
| Isomalt | 94.0 | 94.0 | 94.0 |
| Xylitol | 2.40 | 2.40 | 2.40 |
| Vanillin/Vanillylvanillat | 0.10 | 0,15 | 0.20 |
| Zitronensäure | 0.050 | 0.050 | 0.050 |
| Kirscharoma | 0.25 | 0.25 | 0.25 |
| Wasser | Ad 100 | | |

## Patentansprüche

1. Stoffgemische, enthaltend
(a) Vanillin und
(b) Vanillylvanillat
wobei die Stoffgemische die Komponenten (a) und (b) im Gewichtsverhältnis von etwa 25:75 bis etwa 75:25 enthalten.

2. Gemische nach Anspruch 1, **dadurch gekennzeichnet, dass** sie die Komponenten (a) und (b) im Gewichtsverhältnis von etwa 40:60 bis etwa 60:40 enthalten.

3. Stoffgemische nach mindestens einem der Ansprüche 1 bis 2, **dadurch gekennzeichnet, dass** sie einen Wassergehalt von weniger als 2 Gew.-% aufweisen.

4. Stoffgemische nach mindestens einem der Ansprüche 1 bis 3, **dadurch gekennzeichnet, dass** sie als Kapseln vorliegen.

5. Zubereitungen für die orale Aufnahme enthaltend die Stoffgemische des Anspruchs 1.

6. Zubereitungen nach Anspruch 5 **dadurch gekennzeichnet, dass** sie die Stoffgemische in Mengen von etwa 0,01 bis etwa 1 Gew.-% enthalten.

7. Zubereitungen nach den Ansprüchen 5 und/oder 6, **dadurch gekennzeichnet, dass** es sich um Backwaren, Getränke, Kaugummis, Süßwaren oder Mund- und Zahnpflegemittel handelt.

8. Verwendung von Stoffgemischen nach Anspruch 1 als Aromastoffe.

9. Verwendung nach Anspruch 8, **dadurch gekennzeichnet, dass** man die Stoffgemische in Mengen von etwa 0,01 bis etwa 1 Gew.-% - bezogen auf die konfektionierten Endprodukte - einsetzt.

## Claims

1. Substance mixtures, comprising
(a) vanillin and
(b) vanillyl vanillat
wherein the substance mixtures comprise the components (a) and (b) in a weight ratio of about 25:75 to about 75:25.

2. Mixtures according to claim 1, **characterized in that** the mixtures comprise components (a) and (b) in a weight ratio of about 40:60 to about 60:40.

3. Mixtures according to at least one of claims 1 to 2, **characterized in that** the mixtures comprise a water amount of less than 2% per weight.

4. Mixtures according to at least one of claims 1 to 3, **characterized in that** the mixtures are present as capsules.

5. Preparations for oral intake comprising the substance mixtures according to claim 1.

6. Preparations according to claim 5, **characterized in that** the preparations comprise the substance mixtures in an amount of about 0.01 to about 1% per weight.

7. Preparations according to claims 5 and/or 6, **characterized in that** the preparations are baked goods, beverages, chewing gum, confectionery or oral and dental care products.

8. Use of substance mixtures according to claim 1 as aromatic substances.

9. The use according to claim 8, **characterized in that** the substance mixtures are used in amounts of about 0.01 to about 1% per weight - related to the finished products.

## Revendications

1. Mélanges de matières, contenant
(a) de la vanilline et
(b) du vanillate de vanillyle
les mélanges de matières contenant les composants (a) et (b) en un rapport pondéral d'environ 25:75 à environ 75:25.

2. Mélanges selon la revendication 1, **caractérisés en ce qu'**ils contiennent les composants (a) et (b) en un rapport pondéral d'environ 40:60 à environ 60:40.

3. Mélanges de matières selon au moins l'une des revendications 1 et 2, **caractérisés en ce qu'**ils présentent une teneur en eau inférieure à 2 % en poids.

4. Mélanges de matières selon au moins l'une des revendications 1 à 3, **caractérisés en ce qu'**ils sont présents sous forme de capsules.

5. Compositions pour l'absorption orale contenant les mélanges de matières de la revendication 1.

6. Compositions selon la revendication 5, **caractérisées en ce qu'**elles contiennent les mélanges de matières en des quantités d'environ 0,01 à environ 1 % en poids.

7. Compositions selon les revendications 5 et/ou 6, **caractérisées en ce que** ce sont des produits de boulangerie, des boissons, des chewing-gums, des confiseries ou des produits de soins de la bouche et dentaires.

8. Utilisation de mélanges de matières selon la revendication 1 en tant qu'arôme.

9. Utilisation selon la revendication 8, **caractérisée en ce que** l'on utilise les mélanges de matières en des quantités d'environ 0,01 à environ 1 % en poids par rapport aux produits finis confectionnés.
